# EUROPEAN PATENT APPLICATION

(11) **EP 1 593 375 A1**
(43) Date of publication of application: **09.11.2005**
(21) Application number: 03777416.3
(22) Date of filing: 09.12.2003
(51) Int. Cl.: A61K 9/12, A61K 45/00, A61P 1/10, A61M 3/00

(54) **BODY CAVITY WASHER, DEVICE FOR WASHING BODY CAVITY AND METHOD OF WASHING BODY CAVITY**

(30) Priority: 09.12.2002 JP 2002383148
(71) Applicant: The Mollennium Laboratories, Nisshin-shi Aichi 470-0126 (JP)
(72) Inventor: KUNOGI, Mahito, Nagoya-shi, Aichi 466-0838 (JP); KOIDE, Masafumi, Nagoya-shi, Aichi 468-0051 (JP); YOSHIDA, Hitoshi, Chikusaku, Nagoyashi, Aichi 464-0015 (JP)
(74) Representative: Thoma, Michael
(86) International application number: PCT/JP2003/015747
(87) International publication number: WO 2004/052338

(57) **Abstract**

Mulling contents of an intestine 12 and a gas-liquid mixture is promoted to increase a contact area between adventitious moisture and detained feces at an increasingly fast rate, thereby fluidizing the detained feces. The use of a liquid component and a gas component in combination enables development of an adventitious liquid component to the entire intestine while discomfort and ache are suppressed. As a result, increase in total excretion amount of liquefied detained feces is reduced. Injection and discharge control of the adventitious component enables coping with various defecation impairment.

## Description

### Technical Field

The present invention relates to an organic lumen cleaning agent, an organic lumen cleaning apparatus, and an organic lumen cleaning method, and particularly relates to measures for improving and preventing defecation disorder caused due to dysfunction of an intestine as an organic lumen.

### Background Art

As disclosed in Japanese Patent Application Laid Open Publication No. 11-319082A, stubborn defecation impairment and the like have been conventionally tackled by administration of a laxative, insertion of a gaseous intestine stretching agent, and glycerin enema. In the case where these treatments are insufficient, warmed water is injected and circulated in the intestine by pressure, softening and fluidizing feces to invite defecation.

### -Problems that the Invention is to Solve-

However, the conventional intestine cleaning methods involve a problem that dispersion of detained feces by contact between adventitious moisture and intestinal feces is restrained by symptoms of dysfunction such as contraction of the intestine itself and dyskinesia or of discomfort caused due to increase in pressure within the intestine. As a result, fluidization and excretion of the detained feces hardly reach a predetermined amount for satisfaction in many cases even after a given period of time passes.

Further, a large amount of wormed water injection is required for developing the moisture into the entirety of the intestine. Under the circumstances, in the conventional intestine cleaning method, a mass of excrement from the intestine including the adventitious moisture must be treated. As a result, the conventional intestine cleaning methods involve a large-scale liquid feeding mechanism and a waste water treating mechanism.

The present invention has been made in view of the above problems and aims at coping with various kinds of defecation impairment and the like.

### Summary of the Invention

The present inventors have studied and examined defecation disorder over the years to find the fact that hardened feces can be liquefied in such a manner that a contact area between a liquid component and detained feces increases at an increasingly fast rate by applying a gas-liquid mixture, thus achieving the present invention.

### <Principal Concept>

In the present invention, mulling of a gas-liquid mixture and contents of an organic lumen is promoted within the organic lumen to increase a contact area between an adventitious liquid component and the contents at an increasingly fast rate, thereby liquefying the hardened contents.

For example, in the present invention, mulling of a gas-liquid mixture and contents of an intestine is promoted within the intestine to increase a contact area between an adventitious liquid component and the detained feces at an increasingly fast rate so that the hardened feces is liquefied.

The present invention directs manly to an intestine as the organic lumen but the organic lumen in the present invention includes: nasal cavities; auricular cavities; oral cavities; tracheae; bronchi; esophagi; stomachs; duodena; small intestines; gallbladders; bile ducts; pancreatic ducts; myelonic ducts; subdural cavities; pleuroperitoneal cavities; pericardial cavities; mediastinal cavities; renal pelvises; urethras; vesicourethral canals; uterovaginal cavities; joint cavities; abscess cavities; infection focuses; cysts; blood vessels; and the like. The case where an intestine is directed to as an organic lumen will be described below.

In short, a gas component is used in addition to a liquid component to decrease an administration amount of the liquid component to the intestine. As a result, the adventitious liquid component can develop into the entirety of the intestine while discomfort and ache caused due to increase in pressure in the intestine are prevented. Whereby, increase in total amount of excrement containing liquefied detained feces is reduced.

Further, fluidization of the contents of the intestine is promoted by allowing the gas-liquid mixture to be in a foam state and mechanical injection and excretion of the adventitious component are controlled while local or abrupt increase in pressure in the intestine is prevented, thereby enabling to cope with various defecation impairment.

Moreover, as the gas component as a developing medium of the gas-liquid mixture, a gas that is inert and invites no vascular occlusion is favorable to be used. For example, carbon dioxide (CO₂) is listed. As a source of CO₂, dry ice, CO₂ generated by a chemical reaction or an exothermic reaction, a CO₂ cylinder, a CO₂ spray can may be listed.

Furthermore, an accelerator for fluidizing detained feces as contents of an intestine may be: fluidizing agents harmless to human; various kinds of polysaccharides; rubbers; sugar ester; fatty ester; salts; amino acids; peptide; protein; and aggregate solutions thereof.

Further, a safety holding mechanism (escape) may be accompanied for adjusting an introduction pressure and an introducing amount of the liquid component as a developing medium and the gas component to be introduced into the intestine.

Moreover, the gas-liquid mixture causes mulling of the detained feces in the intestine and the fluidizing agent and causes liquidation of the detained feces to promote physical dispersion of the detained feces in the intestine.

Furthermore, an intestine cleaning apparatus as an organic lumen cleaning apparatus may includes a controller having a path selection switch for controlling defecation and inflow of CO₂ and the fluidizing agent to the intestine.

Further, the intestine cleaning apparatus may include a deodorization/exhaust system for canceling odor from an excreted gas. A container for separating and collecting the gas component and liquefied feces thus excreted may be provided also.

Moreover, the intestine cleaning apparatus may be constituted by a tool or a disposable package that is to be used only one time, or by an apparatus that can be used plural times.

Furthermore, the intestine cleaning apparatus may have a monitor record of apparatus operation for setting optimum operation conditions for individuals. The intestine cleaning apparatus may also have time monitor recording system as a progressive pacemaker of an operation process. For plural-time treatment, a past suitable condition is desirable to be referenced.

In addition, referring to installation location of the intestine cleaning apparatus, hospitals, clinics, aesthetic salons, exclusive service stations, and the like are listed. A mobile intestine cleaning apparatus may be of on-vehicle type or portable type to be used for at-home caregiving and routine visit to group home, and the like. These services may be local-networked and a hand-kit type intestine cleaning apparatus is used in individual home.

### <Concrete Means of Solving the Problems>

An organic lumen cleaning agent according to the first invention includes: a gas-liquid mixture obtained by mixing at least a gas component and a liquid component.

In an organic lumen cleaning agent according to the second invention, the gas-liquid mixture is in a vapor state that the liquid component floats in the gas component.

In an organic lumen cleaning agent according to the third invention, the gas-liquid mixture is in a foam state that the gas component is encapsulated in the liquid component.

In an organic lumen cleaning agent according to the fourth invention, the gas-liquid mixture has a property of fluidizing or dispersing contents of an organic lumen.

In an organic lumen cleaning agent according to the fifth invention, the gas component of the gas-liquid mixture is a gas including at least one of air, oxygen, nitrogen, carbon dioxide, argon, and helium, and the liquid component of the gas-liquid mixture is a liquid of any of an aqueous solution, an oil-based liquid, a water- and oil-based liquid, and a mixed solution of a water-based material and an oil-based material.

In an organic lumen cleaning agent according to the sixth invention, a solute component of the gas-liquid mixture is at least one selected from the group of: celluloses; uronic acids; starches and starch-derived products; dextrins; lactobacillary yogurt and yogurt-derived products; mucopolysaccharides; rubbers; polyvinyl alcohol; polyvinyl pyrrolidone; sugar ester; polyglycerol ester; polyethylene glycol; glycerin; kudzu; seaweeds and seaweeds-derived products; natural oils and synthetic oils; detergents; herbal aromatic substances; natural bactericides and synthetic bactericides; deodorizers; oligosaccharide and monosaccharides; electrolytes; inorganic alkalis; and carboxylic acids.

In an organic lumen cleaning agent according to the seventh invention, the gas-liquid mixture includes a CO₂ generating agent and at least one of bacteria, bacteria-derived products; and carboxylic acids.

In an organic lumen cleaning agent according to the eighth invention, the gas-liquid mixture contains at least one of a biological origin and a physiological modifier.

An organic lumen cleaning apparatus according to the ninth invention includes: insertion means to be inserted into an organic lumen; and cleaning agent means, which stores an organic lumen cleaning agent composed of a gas-liquid mixture obtained by mixing at least a gas component and a liquid component or which generates an organic lumen cleaning agent from a raw material component of the gas-liquid mixture, for supplying the organic lumen cleaning agent to the insertion means.

An organic lumen cleaning apparatus according to the tenth invention further includes: discharge means connected to the insertion means for recovering contents of the organic lumen which is excreted from the organic lumen.

In an organic lumen cleaning apparatus according to the eleventh invention, the cleaning agent means includes volume control means for controlling a supply amount of the organic lumen cleaning agent.

In an organic lumen cleaning apparatus according to the twelfth invention, the cleaning agent means includes a flexible container, and a division wall which is provided within the container, which defines the inside of the container into plural, and which is capable of being broken.

In an organic lumen cleaning apparatus according to the thirteenth invention, the gas-liquid mixture is in a foam state that the gas component is encapsulated in the liquid component.

An organic lumen cleaning method according to the fourteenth invention which uses: insertion means to be inserted into an organic lumen; and cleaning agent means, which stores an organic lumen cleaning agent composed of a gas-liquid mixture obtained by mixing at least a gas component and a liquid component or which generates an organic lumen cleaning agent from a raw material component of the gas-liquid mixture, for supplying the organic lumen cleaning agent to the insertion means, the method including the steps of: inserting the insertion means into an organic lumen; injecting the organic lumen cleaning agent from the cleaning agent means through the insertion means.

In an organic lumen cleaning method according to the fifteenth invention further includes the step of: recovering contents of the organic lumen which is excreted from the organic lumen to discharge means connected to the insertion means after cleaning by injecting the organic lumen cleaning agent into the organic lumen.

In an organic lumen cleaning method according to the sixteenth invention, a supply amount of the organic lumen cleaning agent is controlled by volume control means connected to the connection means.

In an organic lumen cleaning method according to the seventeenth invention, the gas-liquid mixture is in a foam state that the gas component is encapsulated in the liquid component.

An organic lumen cleaning method according to the eighteenth invention further includes the step of: administering an antifoaming agent to the organic lumen in a cleaning process after injecting the organic lumen cleaning agent to the organic lumen.

### <Functions and Effects>

In the present invention, an organic lumen is cleaned by means of the above constitution. Wherein, the cleaning of an intestine will be described.

In general, defecation impairment participates in contraction of an intestine, hypokinesis, flowability lowering and solidification of feces in an intestine, and the like. Actually, a moisture content is 70% or more even in hardened feces and 80 % or more moisture content causes cathartic feces. In contrast, soil liquefaction observed at earthquake requires no additional moisture. Taking account of it, it is important for excretion of detained feces to allow the feces to be in a condition capable of being excreted easily by providing an opportunity for fluidization to hardened feces with no so much moisture added. Further, it is effective to provide physical opportunity for contraction to an smooth muscle of the intestine by intestine stretch and dilation by increasing the contents of the intestine and to allow lubricating molecules to enter into a gap between the intestine wall and feces.

In the present invention, a gas-liquid mixture made of a gas component and a liquid component is used. This gas component may be in a vapor state. Injection of the gas-liquid mixture into the intestine stretches the intestine and dissociates feces from the wall of the large intestine. Further, liquefaction and deposition of the moisture and the like or a material contained in the gas-liquid mixture allow the feces to slide or to be excreted easily. Further, the intestine stretch stimulates the locomotor function of the intestine to promote excretion of the detained feces. Specifically, the liquefaction and the deposition promote easy defecation by allowing a wax, silicon, and the like to enter into a gap between the contents of the intestine and the intestine wall and to be deposited on the interface, promoting distraction of the surface of the feces by introducing gas molecules and their cluster to which high kinetic energy is provided.

In general, no anatomical difference in intestine structure is observed between persons having normal defecation and persons in constipation and there is ignorable difference in moisture content between cathartic feces and hardened feces. Accordingly, in order to excrete detained feces, it is effective to allow hardened feces and the like to be in an easily excreted state by providing an opportunity for fluidization without much moisture added, or providing a physical opportunity for contraction of the smooth muscle of the intestine by stretch and dilation of the intestine by increasing the contents of the intestine.

In the present invention, a gas-liquid mixture is injected into the intestine to stretch the intestine, dissociating the feces from the wall of the large intestine. Further, a fluidizing component and the like contained in the gas-liquid mixture cause the detained feces to be in an easily sliding state and be excreted easily, and volume expansion stimulates the locomotor function of the intestine to promote excretion of the feces. For example, stretch of the large intestine by injection of the liquid and the gas into the large intestine, dissociation of the detained feces from the wall of the intestine, fluidization of the detained feces, and activation of the intestine motion promote excretion of the detained feces. Compared with a case using only a liquid, excessive increase in inner pressure of the intestine can be prevented when the liquid and the gas are injected in combination, resulting in prevention of ache and discomfort and ensuring safety treatment.

In the third invention, the intestine stretches by injecting the foam-state gas-liquid mixture to dissociate the feces from the wall of the large intestine. Further, in cleaning the intestine with the foam-state gas-liquid mixture, a large amount of gas-liquid mixture is distributed in the intestine by slight increase in pressure, compared with the case using only a liquid. This brings safety and efficient stretch of the large intestine, dissociation of the contents (detained feces) from the intestine wall, dispersion and fluidization thereof, and motion activation of the intestine, to lead the detained feces to be excreted. In addition, the gas-liquid mixture in the foam state attains uniform and wide distribution of the fluidizing agent.

When a solution having high viscosity or of which surface tension is small encapsulates the gas, the resultant gas-liquid mixture is easy to be in a foam state.

Further, the gas-liquid mixture is formed outside the body by mixing a solution and a gas under atmospheric pressure or a gas compressed in a gas cylinder. Also, by releasing the gas into the solution, a gas-liquid mixture containing foam is formed in a closed lumen.

Moreover, the gas-liquid mixture may be stored by compressing the gas and the liquid in a cylinder.

Furthermore, the gas-liquid mixture is formed by mixing a solution and a substance in a liquid phase or a solid phase, of which molecules are in a gaseous phase at normal temperature under atmospheric pressure. Also, a substance in a solid phase is put in a solution to form the gas-liquid mixture containing foam by carburetion or sublimation.

Further, the gas-liquid mixture is formed by shaking the gas and the liquid.

Still further, the gas-liquid mixture is formed by producing the gas in the solution by a physical and chemical reaction.

Moreover, the gas-liquid mixture is formed by mulling the solution and the gas generated by heating or combustion.

Furthermore, the gas-liquid mixture is formed by adding the gas to the liquid injected in the intestine.

Further, the gas-liquid mixture is formed by promoting gas release by a chemical reaction in the liquid in the intestine.

Moreover, the gas-liquid mixture is formed by supplying the liquid to a chemical reaction substrate in the intestine which causes gas release.

Furthermore, the gas-liquid mixture is formed by mulling by adding the liquid to the gas injected in the intestine.

Mere mulling of a liquid and a gas results in separation between the gaseous phase and the liquid phase.

When a gas is encapsulated by a liquid that maintains the tension for encapsulating the gas, foam in a state that the inside pressure of the foam is antagonistic to the liquid tension is formed. For example, foam is formed by blowing a gas into a liquid as if soup bubbles are formed.

Further, various schemes are contemplated for mulling the gas and the liquid, namely: the gas is introduced through a nozzle; the gas is continuously generated; a mixing chamber for introducing both the gas and the liquid is employed; and net-like filtering mechanism that causes the gas-liquid mixture to be refined is employed.

The moisture content of even hardened feces is 70 % or more. Therefore, for excreting detained feces, it is important to make the hardened feces and the like to be in an easily excreted state with little amount of moisture added by providing an opportunity for fluidization to the hardened feces. In the fourth invention, a substance having property for promoting specific or non-specific dispersion or fluidization of a substance in the intestine is administered. As a result, increase in pressure in the intestine is suppressed and treatment that brings improvement, prevention or mitigation of constipation is enabled, with the use of less amount of solution.

Further, in the fifth invention, gaseous-phase elements or molecules of any of air, oxygen, nitrogen, carbon dioxide, argon, and helium, and the gaseous mixture thereof is used as the gas component. In addition, in introducing into the intestine, the gas-liquid mixture is preferable in an inert state that hardly causes a chemical reaction and affects less influence to organic cells. Further, carbon dioxide that is high in blood solubility and invites less danger of an embolus is preferable. On other hand, for promoting fluidization of the detained feces, any of solutions, oil-based liquids, water- and oil-based liquids, and a mixed solution of a water-based material and an oil-based material may be used only if it can become in a foam state for rapid development in the intestine. Especially, a substance having high biocompatibility, such as celluloses including methylcellulose that increases viscosity of a solution, polyvinyl alcohol, and emulsifiers such as sugar ester that provides smoothness, is preferably contained. Natural soup and synthetic soup that cause no damage to mucous membranes are preferable, also.

Moreover, in an actual enema treatment, fluidity, smoothness, and especially, acceptability to human sensation are required separately from foam formation. Accordingly, it is useful to use, as a component, a refreshing, clean, and deodorant substance presenting functions of bacteriostasis disinfection and intestine regulation in combination.

In the sixth invention, any of or a combination of methylcellulose, pectin, dextrins, guar gum, polyvinyl alcohol, sugar ester, polyglycerol ester, polyethylene glycol, glycerin, kudzu, starch and starch-derived products, natural or synthetic oils such as palm oil, castor oil, and rape oil, surfactants such as tyloxapol, various kinds of detergents, aromatic substances such as menthol, mint, spearmint, lavender, phyton, and cedrol, bactericides such as hinokitiol, deodorant adsorbents such as activated carbons, oligosaccharide, monosaccharide, and lactobacillus preparations are preferable as a solute component.

Moreover, the solute component may be inorganic alkalis such as baking soda, or carboxylic acids. The carboxylic acids include monocarboxylic acids such as lactic acid, dicarboxylic acids such as tartaric acid, and tricarboxylic acids such as citric acid.

For example, one of herbs, milk thistle enhances detoxification in livers and burdock is used for assisting function enhancement of kidneys and levers. They may be used solely or in combination of two or more appropriately. Among of all, basically, combination with methylcellulose and the like and ester preferably promotes foam formation and mulling and fluidization of the detained feces.

Furthermore, compounding ratio of, for example, methylcellulose is preferably in the range between 0.01 and 5 w/v % normally in the composition, that of cyclodextrin is preferably in the range between 0.001 and 5 w/v % normally in the composition, that of polyvinyl alcohol is preferably in the range between 0.01 and 10 w/v % normally in the composition, and that of sugar ester is preferably in the range between 0.1 and 5 w/v % normally in the composition.

If the compound ratio of the above polymer compounds is too small, wettabilty of the feces in the intestine is insufficient, and accordingly, fluidization of the stayed feces may be insufficient. In addition, the compound ratio of the above polymer compounds is too large, the viscosity becomes so high to cause a feeling of wrongness in injection operation to the intestine.

In the present invention, in addition to the above listed components, the aforementioned medicines for external use for enema, ointments, and various buffering agents generally used in preparation such as gels, various kinds of additives such as solution adjuvant, tonicity agents, stabilizers, thickening agents, chelating agents, pH adjusters, and algefacients, and other medically effective components may be compounded within a range that does not inhibit the effects of the present invention.

Specifically, the buffering agents include: boric acid or its salts (borax and the like); citric acid or its salts (sodium citrate and the like); phosphoric acid or its salts (monosodium phosphate and the like); tartaric acid or its salts (sodium tartrate and the like); gluconic acid and its salts (sodium gluconate and the like); acetic acid and its salts (sodium acetate and the like); lactic acid and its salts (sodium lactate and the like); various kinds of amino acids; and combinations thereof.

The solution adjuvant includes a small amount of polyethylene glycol, propylene glycol, and the like.

The tonicity agents include: sodium chloride; potassium chloride; mannitol; propylene glycol; and the like, for example.

The stabilizers include: disodium edetate; cyclodextrin; sulfite; citric acid and its salts; and the like, for example.

The thickening agents include: polyethylene glycol; polyvinyl alcohol; polyvinyl pyrrolidone; hydroxyethyl cellulose; hydroxylpropyl methylcellulose; methylcellulose; chondroitin sulfate sodium; and the like, for example.

The chelating agents include: disodium edetate; sodium citrate; and the like, for example.

The pH adjusters include: hydrochloric acid; citric acid and its salts; boric acid and its salts; phosphoric acid and its salts; acetic acid and its salts; tartaric acid and its salts; sodium hydroxide; potassium hydroxide; sodium carbonate; sodium hydrogen carbonate; and the like, for example.

The algefacients include: menthol; borneol; camphor; geraniol; limonene; eugenol; peppermint oil; eucalyptus oil; and the like, for example.

The intestine cleaning agent in the present invention is normally within the range between pH 4 and pH 9. The administration amount is not limited specifically only if it is within the range that is medically allowable. The administration amount of the intestine cleaning agent including CO₂ in the present invention is 30 to 500 ml per time and the agent is preferably administered several times repeatedly.

Supposed that a normative amount of feces of an adult per time is 300 ml, the water content thereof is 70 % in normal feces and about 85 % in cathartic feces, and accordingly, a moisture amount required for fluidizing the feces is 45 ml. With the use of this amount as a reference, air of about 300 ml in the foam-state or in the gas-liquid mixture is injected in the organic lumen, taking account of a subject's age, sex, and health condition. One- to five-minute injection period is appropriate, and accordingly, the injection speed is about 1 to 5 ml/sec.

The pressure of injection into the intestine is normally below 0.5 m of water and not exceeds 1.4 m of water at the maximum. Volume of a gas is inversely proportional to pressure, and accordingly, it takes time to increase pressure unless the injection speed is in error. For example, it takes 5 seconds at injection speed of 1 ml/sec to increase the pressure of 100 ml gas by 0.5 m of water (0.05 atm). The sum of the gas within an injection pipe and the like and the gas of a discharge pipe and the like is over the volume at injection, so that an abrupt pressure increase can be avoided. As a result, the intestine cleaning in the present invention is high in safety, compared with the conventional cleaning methods that use uncompressed liquid.

For cleaning an intestine, an inert gas such as CO₂ is preferably used and environment that brings excellent enterobacterium plexus is preferably maintained. In this connection, the seventh invention mainly introduces baking soda, yogurt product and the like to attain both generation of inert and safe CO₂ and optimum intestinal environment.

In the eighth invention, the component of biological origin and the like is included, enabling administration of the active material and the like to the body. Various food includes bacteria, artificial additives (preservatives, colorants, flavorings), food-related materials (components dissolved from coating paper and the like). Further, solvents in environment and chemicals such as dioxin may enter into a body. The intestine cleaning has no alternative that provides an opportunity of adsorbing and removing poison adhered to the intestine wall and circulated in the intestine and that can provide an opportunity of administering antidotal materials and active materials into the body through mucous membranes. On the other hand, organs such as kidneys, livers, lungs, intestines and the like have functions of activation and poison excretion, and therefore, administration of biologic active materials of various kinds of nucleic acids, amino acids, peptide, and protein through the intestine before and after the intestine cleaning enhances physiological functions of the body together in addition to detoxification. Further, when the intestine is cleaned and stretched, environment suitable for absorbing the active materials and nutrients through the entirety of the mucous membranes of the intestine is realized. Thus, the present invention is not only means effective for the intestine locally but also is one of the most suitable means for conveying materials required for general administration. In addition, the present invention provides an opportunity for enhancement of nutrition, immunity, and physiological activation for persons who can ingest nothing. Almost of all nucleic acids, especially RNA, through peroral administration are subjected to enzyme degradation before exhibiting their function. This problem can be solved by administration to the intestine in the present invention. Further, it is recognized that nucleic acids, amino acids, peptide, protein, and the like have a function of forming foam.

The tenth invention provides the discharge means to promote pleasing defecation. In detail, stretch of the intestine and fluidization of the feces generally cause the detained feces to spontaneously flow out. However, a human body of which function of spontaneous intestine motion is lowered is incapable of spontaneous stretch even if detained feces are fluidized in the intestine. In this case, when discharge means is provided such as vacuum aspiration, repetition of injection and aspiration of the contents of the intestine including the intestine cleaning agent, belly massage, irradiation of ultrasonic wave, defecation can be caused.

When excrement is released upon excretion, odor and waste disposal are problematic. Accordingly, the opening/closing means is preferably provided. It is preferable to recover the excrement to a closed container or to introduce the excrement into a sanitary bowl or a pipe connected to sewage or to a septic tank. This series of equipment may be integrated or provided as several units.

Treatment for distributing a material that causes specific or non-specific fluidization of the intestinal components from the surface of feces in the large intestine into the inside of the feces is effective as treatment for constipation, defecation by a man who is under rest after operation, and various kinds of intestine disorder.

However, in a case of stubborn defecation impairment, distribution impedance of the injected material within the intestine may be caused. Therefore, not only discharge of contents of the intestine but also reflux, jetting, and repetitive injection/discharge of the to-be-injected material, employment of auxiliary means such as oscillator, and the like are performed for promoting distribution within the intestine and fluidization of the stayed feces.

Specifically, distribution of the injected materials to lumps of the detained feces is caused by any one or a combination of manual or automatic pump reflux, screw mulling, pressure reduction and vacuuming, pulse oscillation, ultrasound stimulation, and atomizing, thus promoting dispersion and fluidization.

When the gas-liquid mixture is introduced into the intestine for fluidizing the detained feces, it is appropriate that a subject is in a recumbent position or in a lateral decubitus position. In that time, if a subject can do spontaneous excretion, he or she goes to a toilet for normal defecation when he or shi feels the need to defecate. When a subject is disabled or is in an aesthetic salon, equipment, an operator who handles the apparatus or a treatment operator does defecation and disposal of the excrement in lieu to the subject. If the excreted feces are spread out around the periphery, odor and burden to disposal are problematic. The tool or the apparatus for introducing and storing feces excreted by reflux or the discharge means through a conduit is operated to perform disposal to a closed section or introduction to swage.

For introducing the gas-liquid mixture into the intestine for fluidization of the detained feces, it is convenient that a subject is in the recumbent position or a lateral decubitus position. If it is difficult to stimulate the intestine motion of a subject who is in either position or if a subject is disabled or in a aesthetic salon, additional means for further fluidization of the detained feces and defecation is necessary. Accordingly, the gas-liquid mixture is sent and received in the outside of the body connected to the intestine to promote dispersion and fluidization of the contents within a combined circuit including the intestine. Further, disposal is important and is effective if defecation and storage of excrement is done by the tool or an apparatus operator. If excreted feces are spread out around the periphery, odor and burden to disposal are problematic. When the excreted feces are retained in the closed lumen, a disposal container is connected through the conduit and the tool or the apparatus for storing feces excreted by reflux are operated, clean treatment can be attained. Absorption of the gas component and separation of the liquid component by release into the air decrease the amount of the excrement.

In the eleventh invention, the supplied amount is controlled by the volume control means. For example, a small amount of administration, vacuuming, a larger amount of administration, less vacuuming from the intestine, and so on are performed in this order by means for causing steady flow, spiral flow, jetting, or pulsation flow, means for reducing pressure to a predetermined value, or volume setting means, rather than one-time injection of the adventitious substance into the intestine. Whereby, the injected material is filled in the intestine gradually. These processes exhibit an effect of slow dilation of the intestine to easily flow the detained feces even in stubborn constipation.

Furthermore, for example, after the contents of the intestine is fluidized by stretching and dilating the intestine by the foam-state gas-liquid mixture, the feces are excreted outside the body, and then, the solution to be injected of which main component is an electrolyte is injected and excreted again. In order that each component functions effectively in the process series for developing the organic function ameliorating substance and the algefacient in the intestine, it is required to optionally set the amount and speed of injection, the number of repetition, and the sequence. Therefore, the components separated in several compartments are blended according to individuals and used, to attain tailor-made intestine cleaning.

The twelfth invention provides a simple apparatus. For example, the compressed gas and the liquid are stored separately in an easily-broken container which is made of plastic, rubber, or the like and which is partitioned by the division wall. The breakage of the division wall causes the gas-liquid mixture to be generated. When the tip end of the container is inserted and released in the rectum in advance, the gas-liquid mixture is developed into the intestine to fluidize the detained feces.

The constitution for mulling the gas and the liquid by integration of two parts of a housing unit for the material for gas generation and a housing unit for the liquid may be employed. When the gas-liquid mixture is generated in a folded bag, a sufficient amount of the substance to be injected into the intestine can be obtained within the bag expanded at the gas generation. Accordingly, a user may adjust the injection amount to the intestine by bag compression while taking account of his or her feeling of fullness. The injection material contains a nonionic surfactant, a viscous material, and a fluidizing material, causing the feces to be fluidized. When the injected substance container is in the expandable form, it can be used for housing both the injected substance material and the excrement excreted by vacuum and expansion.

In the eighteenth invention, the antifoaming agent is injected to facilitate the disposal after excretion. When the injected material for intestine cleaning once fluidizes the detained feces, foam is no longer necessary for excretion invitation. When the antifoaming agent is used to separate the gas and liquid components, the foam is removed and the contents of the intestine become in the state that it is easily separate from the gas, facilitating disposal after excretion of the contents of the intestine. Thus, the use of the antifoaming agent is determined taking account of the subject's feeling of fullness under the intestine cleaning treatment.

The excrement from the intestine and cells or components derived therefrom may be evaluated as a sample, or cytoplasm evaluation and gene sequence evaluation utilizing the material to be injected in the intestine may be performed. Fecal examination is performed for various kinds of evaluations for intestines. However, various food-derived contaminants inhibit accurate and sensitive evaluation. The contaminants lower both sensitiveness and specificity in, for example, human hemoglobin detection. Employment of test sample subjected to the cleaning according to the present invention, and employment of a scheme of filtering, centrifuge separation, amplification of nucleic acids, combination reaction, enzyme reaction, and the like using the test sample enable provision of the sample and of opportunity for further accurate and sensitive cell function evaluation and genotypic identification. In addition, when the mucous membranes in the intestine include a deteriorated part, a material having a combining ability to the deteriorated part and a blocking agent are injected to identify the deteriorated part by an endoscopic method through a chemical reaction of a phosphor, an isotope, enzyme, or the like or to identify it from the outside of the body.

It is preferable at the intestine cleaning that the detained feces are being softened and the intestine is stretched, and it is also preferable after the cleaning that the normal intestine function is maintained. Accordingly, peroral supplement of moisture such as a isotonic liquid, or of fibers, intestine motion accelerators, and the like before and after the intestine cleaning leads to further easy and effective excretion. Belly massage before, during, and after the defecation treatment in combination is effective also.

The apparatus for treating detained feces in the intestine by ultrasonic wave, shock wave, or physical oscillation and the oscillation output terminal may be provided. At foam burst, large shock wave energy is generated locally at a small point. As such, when ultrasonic wave, shock wave, or physical oscillation which cause dispersion and fluidization of the detained feces in the intestine, the detained feces and the aqueous solution, or the foam and the gas-liquid mixture is provided in the intestine or from the outside of the body, the feces is impacted and broken to be easily excreted. Thus, the intestine cleaning exhibits extra effects.

It is possible to provide services including routine visit, on-site services, and the like for providing the intestine cleaning and accompanying treatment, leasing business for leasing the services to a third party, and franchise business that allows a third party to undertake the services.

For example, effective and easy intestine cleaning in home involves problems in manpower, equipment, and skill of an operator. Services for providing the intestine cleaning by the gas-liquid mixture and the accompanying treatment may be performed for providing health maintenance services through home or facility visiting or in a satellite opened regionally.

The conventional intestine cleaning methods injects warm water by pressure, which requires a large-scaled apparatus and injection and discharge pipes and requires arrangement of nurses and the like capable of performing treatment equivalent to medical practice and construction of an exclusive treatment room. The present apparatus is composed of simple and safety components so as to be of vehicle-installed type or portable type, enabling provision of visiting services to individual home and nursing-care facilities.

Even in services provision through apparatus installation, fewer manpower only for monitoring a TV monitoring center and a recording instrument is sufficient, and thus, space-saving and manpower-saving under the condition that the safety is secured is realized, which has been impossible conventionally.

Referring to a concrete example, a space for installing the apparatus or the tool, accessories, and an excretion disposal system including a toilet, a working space for an operator, and the like beside a bed on which a client receiving the services lies in a recumbent position are about several tens of percentages of that in the conventional method. When various treatments are performed in parallel, the gas and liquid components are prepared separately in packages for individuals or in packages in blocks.

Accordingly, there are cases where the gas-liquid supply source and the excretion disposal system are prepared for each of a plurality of subjects and cases where the gas-liquid is supplied uniformly through the pipes to a plurality of subjects and excrement is disposed in batch. To sum up, the system of the present invention enables various kinds of collective development, so that further wide business development and contribution to society are expected by lease and rental of the business system for providing the services and the business for providing the services to a contractor.

### <Effects of the Invention>

As described above, the present invention provides the gas-liquid mixture of the gas component and the liquid component. Accordingly, mitigation of burden and safety and positive defecation can be provided to persons in defecation impairment caused due to various factors such as bedridden persons who are failure to intestine motion, persons after operation, and young to middle-age females in constipation. The defecation impairment invites anorexia due to feeling of fullness and serves as a trigger for various bad conditions to lower body force and resistance. However, the present invention enables not only the intestine cleaning but also effective injection of the various kinds of effective components to the intestine, contributing improvement in the organic function.

### Brief Description of the Drawings

FIG. **1** is a schematic section showing a constitution of an intestine cleaning apparatus according to Working Example 1 of the present invention.
FIG. **2** is a flowchart depicting cleaning operation in Working Example 1.
FIG. **3** is a schematic section showing a constitution of an intestine cleaning apparatus according to Working Example 2 of the present invention.
FIG. **4** is a schematic section showing a constitution of the intestine cleaning apparatus according to Working Example 2 at excretion of contents of an intestine.
FIG. **5** is a schematic diagram showing a constitution of a main part of an intestine cleaning apparatus according to Working Example 3 of the present invention.
FIG. **6** is a schematic diagram showing a constitution of the main part of the intestine cleaning apparatus according to Working Example 3 at injection of an intestine cleaning agent.
FIG. **7(A)** is a front section showing an intestine cleaning apparatus according to Working Example 4 of the present invention, and FIG. **7(B)** is a side section showing the intestine cleaning apparatus according to Working Example 4 of the present invention.
FIG. **8** is a flowchart depicting a cleaning process using the intestine cleaning apparatus of Working Example 1.
FIG. **9** is a list indicating properties of intestine cleaning agents in Embodiment 1.
FIG. **10** is a list indicating properties of intestine cleaning agents in Embodiment 2.
FIG. **11** is a list indicating properties of intestine cleaning agents in Embodiment 3.

### Best Mode for Carrying Out the Invention

Working Examples of the present invention will be described below in detail with reference to the drawings.

### <Working Example 1>

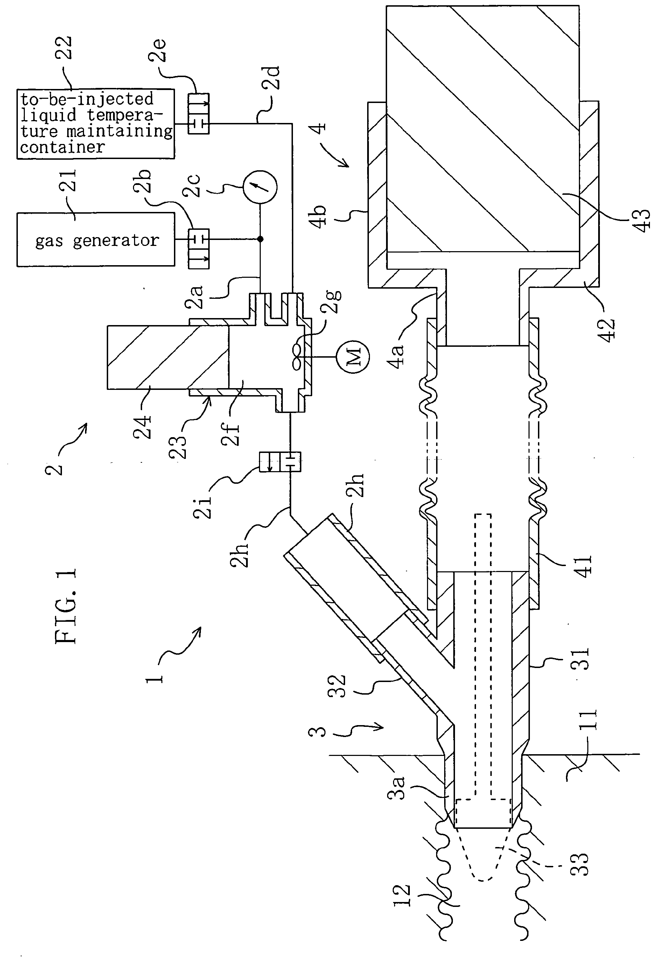

As shown in FIG. **1,** an intestine cleaning apparatus **1** as an organic lumen cleaning apparatus is an apparatus for cleaning an intestine **12** in a body **11** by injecting an intestine cleaning agent as an organic lumen cleaning agent into the intestine **12.** The intestine cleaning apparatus **1** includes a supply mechanism **2,** an insertion jig **3,** and a discharge mechanism **4.**

The supply mechanism **2** composes cleaning agent means for generating the intestine cleaning agent of a gas-liquid mixture. The supply means **2** includes a gas generator **21,** a cleaning agent container **22,** and a mixer **23.**

The gas generator **21** generates and stores a gas component of the gas-liquid mixture. As the gas component, CO₂ (carbon dioxide) is listed for example. The gas generator **21** is composed of a CO₂ cylinder or the like so as to be connected to the mixer **23** through a conduit **2a.** To the conduit **2a,** a closing valve **2b** is provided and a pressure gage **2c** is connected. The closing valve **2b** is a valve for allowing the gas component to flow and for blocking the flow and opens and closes the conduit **2a.**

The cleaning agent container **22** stores a liquid component of the gas-liquid mixture. As the liquid component, an aqueous solution is listed for example. The cleaning agent container **22** maintains the intestine cleaning agent at a predetermined temperature, for example, 37 °C. The cleaning agent container **22** is connected to the mixer **23** through a conduit **2d.** To the conduit **2d,** a closing valve **2e** is provided. The closing valve **2e** is a valve for allowing the liquid component to flow and for blocking the flow, services as a measuring section, and opens and closes the conduit **2d.**

The mixer **23** mixes the gas component of the gas-liquid mixture from the gas generator **21** and the liquid component of the gas-liquid mixture from the cleaning agent container **22.** The mixer **23** includes a stirring tool **2g** provided in a stirring space **2f** and generates a foam-state gas-liquid mixture where the gas is encapsulated in the liquid in the stirring space **2f.** The mixer **23** is connected to the insertion jig **3** through a conduit **2h.** To the conduit **2h,** a closing valve **2i** is provided. The closing valve **2i** is a valve for allowing the intestine cleaning agent to flow and for blocking the flow and opens and closes the conduit **2h.** Further, the mixer **23** is provided with control section **24** for controlling pressure and volume. The control section **24** is composed of, for example, a piston and serves as volume control means for controlling injection pressure and an injection amount of the intestine cleaning agent to be injected to the intestine **12.**

The insertion jig **3** is to be inserted to the intestine **12** and includes a cylindrical main pipe **31** and a sub pipe **32** connected to the main pipe **31.** The tip end of the main pipe **31** is thin to form an insertion tube portion **3a** to be inserted in the lumen. The sub pipe **32** is connected at the tip end thereof to the central portion of the main pipe **31** so as to obliquely incline from the rear end toward the tip end of the main pipe **31.** At the rear end of the sub pipe **32,** the mixer **23** is connected through the conduit **2h.** On the other hand, the rear end of the main pipe **31** is connected to the discharge mechanism **4.**

The discharge mechanism **4** composes discharge means for recovering contents of the intestine **12** as excrement from the intestine **12.** The discharge mechanism **4** includes a discharge pipe **41,** a reservoir **42,** and a vacuum section **43.** The discharge pipe **41** is in a corrugated form having one end connected to the rear end of the main tube **31** of the insertion jig **3.** The reservoir **42** includes a small-diameter connection tube **4a** connected to the other end of the discharge pipe **41** and a large-diameter reservoir tube **4b** continuing to the connection tube **4a.**

The vacuum section **43** is provided at the reservoir tube **41** and is composed of, for example, a piston. The vacuum section **43** compellingly recovers the excrement as the contents of the intestine **12** by applying negative pressure to the inside of the reservoir pipe **4b.**

### -Cleaning Method-

Cleaning operation of the aforementioned intestine apparatus **1** and an intestine cleaning method will be described next.

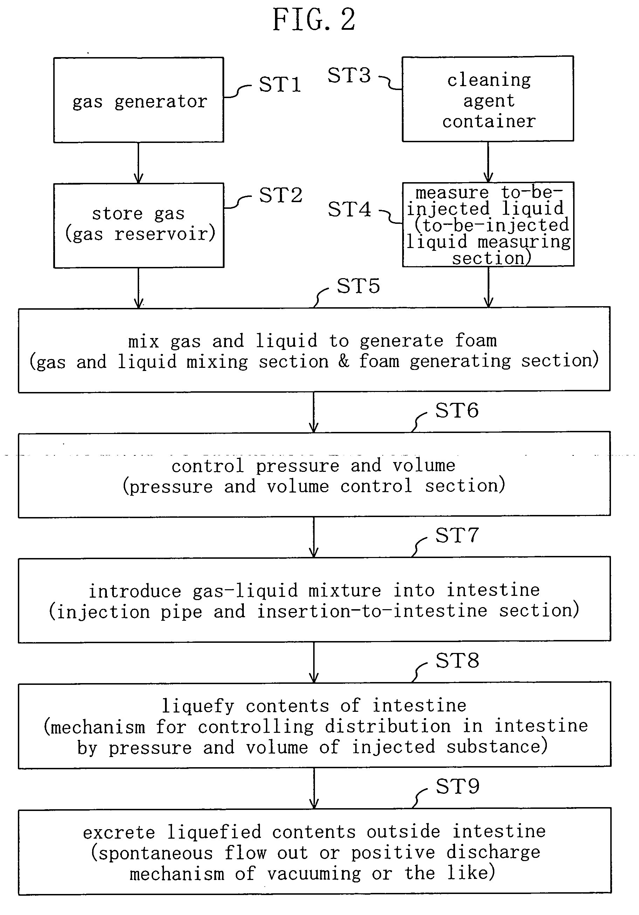

As shown in FIG. **2,** the gas component of the gas-liquid mixture is generated by the gas generator **21** in a step ST1, and the gas component is stored in a step ST2.

On the other hand, in a step ST3, the cleaning agent container **22** stores the liquid component of the gas-liquid mixture maintained, for example, at 37 °C. Wherein, the three closing valves **2b, 2e, 2i** of the supply mechanism **2** are closed in this state.

Thereafter, the insertion jig **3** is inserted into the intestine **12** of the body **11** and the closing valve **2b** of the gas generator **2** and the closing valve **2e** of the cleaning agent container **22** are opened. By this opening of the closing valve **2e,** an injection amount of the liquid component to be injected to the body **11** is measured and the liquid component is supplied to the mixer **23** in a step ST4. At the same time, the gas component of the gas-liquid mixture is supplied to the mixer **23.**

Subsequently, the routine proceeds to a step ST5 in which the liquid component and the gas component of the gas-liquid mixture is stirred by the stirring tool **2g** in the stirring space **2f** in the mixer **23** to generate foam, thereby generating the intestine cleaning agent of the foam-state gas-liquid mixture. Pressure and volume of the intestine cleaning agent are controlled by the control section **24** and the intestine cleaning agent is allowed to flow into the insertion jig **3** through the conduit **2h** by opening the closing valve **2i** of the mixer **23.**

Then, in a step ST7, the intestine cleaning agent flows from the sub pipe **32** to the main pipe **31** of the insertion jig **3** to be injected into the intestine **12.** At that time, as described above, the pressure and the volume of the intestine cleaning agent is controlled by the control section **24.**

The intestine cleaning agent injected in the intestine **12** liquefies the contents of the intestine **12** in a step ST8. Especially, the foam enters between the intestine **12** and the contents to promote fluidization and liquefaction of the contents of the intestine **12.**

Thereafter, the routine proceeds to a step ST9 in which defecation is performed after removing the insertion jig **3** or is performed spontaneously to the discharge pipe **41** mounted to the insertion jig **3** after several minutes to some dozen minutes passes after the injection of the intestine cleaning agent. Alternatively, negative pressure is applied to the reservoir **42** by the vacuum section **43** to cause excretion of the liquefied contents outside the intestine **12.** The excreted contents is recovered in the reservoir tube **4b.**

Wherein, in the case where complete defecation is not attained by one time treatment described as above, the injection amount of the foam-state gas-liquid mixture to be injected to the intestine **12** is increased and the aforementioned operation is repeated to clean the intestine **12.**

Before the treatment is terminated, the foam-state gas-liquid mixture may be flown out by a tonicity agent containing an algefacient.

### -Intestine Cleaning Agent -

The intestine cleaning agent as the gas-liquid mixture used in the intestine cleaning apparatus 1 is preferably obtained by mulling an acid solution (20 ml: 1 % methylcellulose, and 50% supernatant liquor of yogurt, and 10 % citric acid) and baking soda (5 ml of 5 % sugar ester and 12 ml of saturated baking soda solution at 20 °C). Excellent foam of 500 ml is formed slowly. The methylcellulose in the intestine cleaning agent causes foam formation and deceleration of the reaction speed, while the sugar ester assists uniform, excellent, and fine foam formation.

In short, the intestine cleaning agent is formed of the gas-liquid mixture obtained by mixing the gas component and the liquid component. The gas-liquid mixture may be formed of vapor of the liquid component floating in the gas component.

The gas-liquid mixture is preferably in the foam state that the gas is encapsulated in the liquid. The gas-liquid mixture may have a property of fluidizing or dispersing contents of an intestine.

The gas component of the gas-liquid mixture is composed of a gas containing at least one of air, oxygen, nitrogen, carbon dioxide, argon, and helium, while the liquid component of the gas-liquid mixture is composed of a liquid of any of an aqueous solution, an oil-based liquid, a water- and oil-based liquid, and a mixture of an oil-based material and a water-based material.

The solute of the gas-liquid mixture is at least one selected from the group of celluloses, uronic acids, starches and starch-derived products, dextrins, lactobacillary yogurt and yogurt-derived products, mucopolysaccharides, rubbers, polyvinyl alcohol, polyvinyl pyrrolidone, sugar ester, polyglycerol ester, polyethylene glycol, glycerin, kudzu, seaweeds and seaweeds-derived products, natural oils and synthetic oils, detergents, herbal aromatic substance, natural bactericides and synthetic bactericides, deodorizers, oligosaccharide and monosaccharides, electrolytes, inorganic alkalis, and carboxylic acids.

Raw materials of the gas-liquid mixture may include a CO₂ generator and at least one of bacteria, bacteria-derived products, and carboxylic acids.

The gas-liquid mixture may contain at least one of biological origins and organic function modifiers.

### -Effects of Working Example 1 -

As described above, in the present invention, the gas-liquid mixture of the gas component and the liquid component is used, bringing burden mitigation and safety and positive defecation of persons in defecation disorder caused due to various factors, such as bedridden persons in intestine akinesia, persons after operation, young and middle-age females in constipation, and the like. The defecation impairment invites anorexia due to feeling of fullness and serves as a trigger for various bad conditions to lower body force and resistance. However, the present invention enables not only the intestine cleaning but also effective injection of the various kinds of effective components to the intestine, contributing improvement in organic function.

### -Modified Example of Working Example 1-

The insertion tube portion **3a** of the insertion jig **3** may be provided with a tapered guide head **33** as shown in the broken line in FIG. **1.** The guide head **33** facilitates insertion into the intestine **12** and is structured so as to be removed after insertion.

### <Working Example 2>

Working Example 2 of the present invention will be descried next in detail with reference to the drawings.

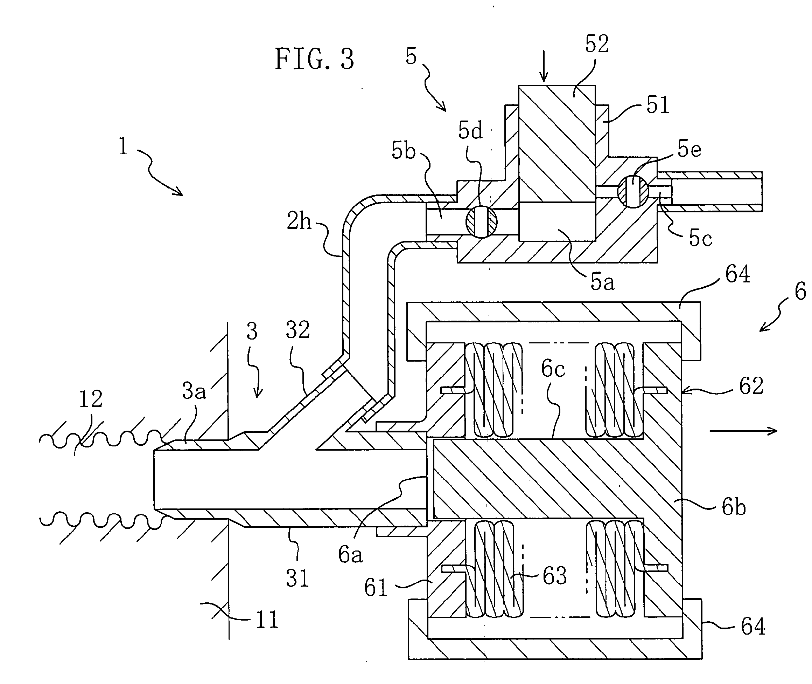

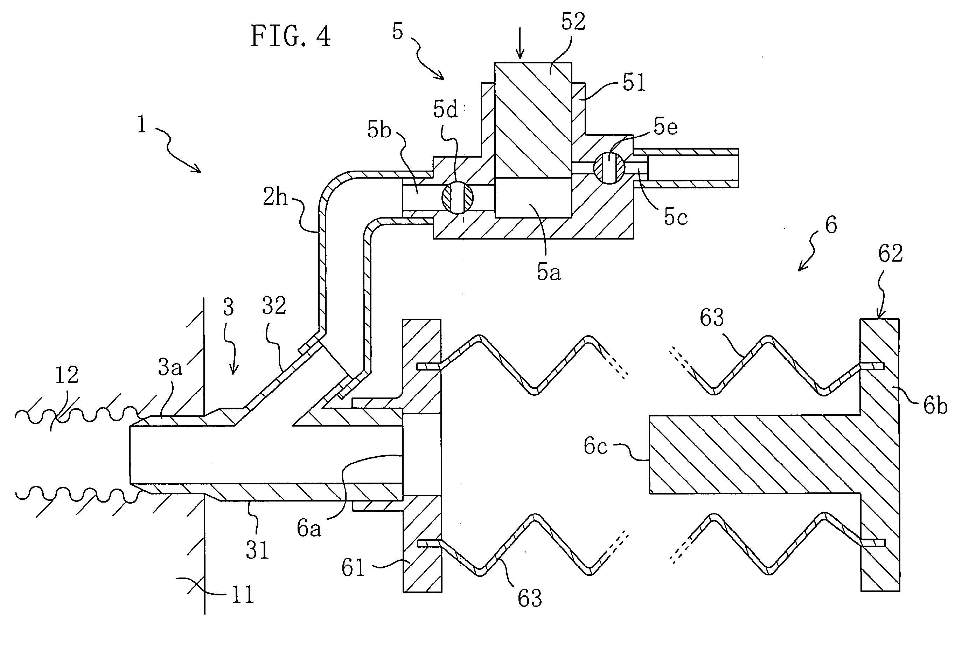

As shown in FIG. **3** and FIG. **4,** a simple supply mechanism **5** is provided rather than the supply mechanism **2** in Working Example 1.

The supply mechanism **5** includes an intestine cleaning agent container **51,** and a piston **52** provided in the container **51.** A housing space **5a** is formed in the upper part of the container **51.** The piston **52** is inserted in the housing space **5a** so as to push out the intestine cleaning agent in the housing space **5a.**

In the container **51,** a supply path **5b** and an air breathing path **5c** which communicate with the housing space **5a** are formed. A closing valve **5d** is provided in the supply path **5b** while an air breathing valve **5e** is provided in the air breathing path **5c.** The supply path **5b** is connected to the insertion jig **3** through the conduit **2h.**

The discharge mechanism **6** connected to the insertion jig **3** is provided with a fixed plate **61,** a tap plate **62,** a corrugated pipe **63,** and a clamp **64.** The fixed plate **61** is fixed at the insertion jig **3** and an opening **6a** corresponding to the main pipe **31** is formed therein. The tap plate **62** includes an end plate **6b** corresponding to the fixed plate **61,** and a plug body **6c** integrally formed with the end plate **6b** and fitted in the opening **6a** of the fixed plate **61.**

The corrugated pipe **63** is connected to the fixed plate **61** and the tap plate **62** so as to recover excrement. Further, the clump **64** fixes the fixed plate **61** and the tap plate **62** in a state that the plug body **6c** of the tap plate **62** is fitted in the opening **6a** of the fixed plate **61.**

### -Cleaning Method-

Cleaning operation of the aforementioned intestine apparatus **1** and an intestine cleaning method will be described next.

First, a liquid component of a gas-liquid mixture containing methylcellulose or the like capable of easily generating foam is introduced under the condition that the piston **52** of the supply mechanism **2** is removed, and dry ice for generating a gas component is put thereinto. Wherein, in this state, the closing valve **5d** and the air breathing valve **5e** are closed.

Next, the piston **52** is inserted into the container **51** and the air breathing valve **5e** is opened to remove surplus air, and then, the air breathing valve **5e** is closed. In so doing, a carbonic acid gas is generated from the dry ice to push up the piston **52.** Then, the gas component and the liquid component are mixed to generate the gas-liquid mixture at a mixed ratio and volume predetermined according to weight measurement.

While, when the closing valve **5d** is opened and the piston **52** is pushed under the condition that the insertion jig **3** is inserted in the intestine **12,** the intestine cleaning agent of the gas-liquid mixture is injected into the intestine **12** through the insertion jig **3.**

During the time when the intestine cleaning agent is injected and a predetermined time period passes, the clump **64** is mounted. Under this condition, the contents of the intestine **12** is fluidized and liquefied but is not excreted.

Thereafter, when the clump **64** is removed, as shown in FIG. **4,** the tap plate **62** retreats to allow spontaneous defecation by inner pressure of the intestine **12,** so that the contents of the intestine **12** is recovered in the corrugated pipe **63.** When the tap plate **62** is forcibly drawn back to extend the corrugated pipe **63** forcibly, the contents of the intestine **12** is sucked and compelled defecation is performed.

The other constitution, operation and effects are the same as in Working Example 1.

### <Working Example 3>

Working Example 3 of the present invention will be described in detail next with reference to the drawings.

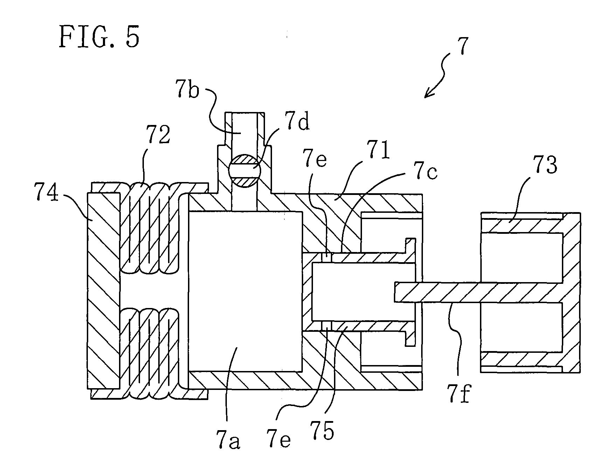

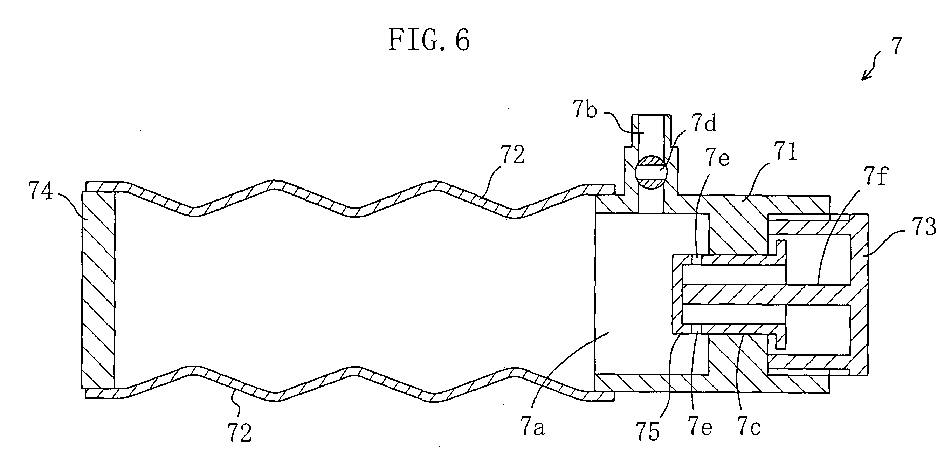

As shown in FIG. **5** and FIG. **6,** another supply mechanism **7** is employed rather than the supply mechanism **2** in Working Example 1.

The supply mechanism **7** includes a mixture container **71,** a corrugated pipe **72,** and a foaming agent container **73.** A mixing space **7a** is formed in the mixture container **71** and the mixture container **71** is connected at an open end of the mixing space **7a** to the corrugated pipe **72.** The end of the corrugated pipe **72** is closed by means of a lid **74.**

In the mixture container **71,** a supply path **7b** communicating with the mixing space **7a** and a foaming agent path **7c** are formed. A closing valve **7d** is provided in the supply path **7b** while a closing plug **75** is fitted in the foaming agent path **7c.** Wherein, the supply path **7b** is connected to the insertion jig **3** through the conduit **2h,** though not shown.

The closing plug **75** is in the form including a hollow of which one end is opened, and a communication port **7e** is formed at the end portion of the closing plug **75** so as to pass therethrough. Further, the closing plug **75** is formed so that the foaming agent container **73** is inserted therein. The foaming agent container **73** is capable of storing the foaming agent and of being inserted in the foaming agent path **7c,** and is provided with a pushing bar **7f** for pushing the closing plug **75.**

### -Cleaning Method-

Cleaning operation of the aforementioned intestine apparatus 1 and an intestine cleaning method will be described next.

First, the closing plug **75** is removed under the condition that the closing valve **7d** of the mixture container **71** of the supply mechanism **7** is closed while the corrugated pipe **72** is contracted, thereby introducing into the mixing space **7a** of the mixture container **71a** predetermined amount of a liquid component of a gas-liquid mixture which contains methylcellulose or the like capable of easily generating foam and which is maintained at about 37 °C. Then, the closing plug **75** is fitted to the foaming agent path **7c** to close the mixing space **7a.**

Subsequently, a predetermined amount of a foaming agent obtained by, for example, mixing powdered tartaric acid and powdered sodium bicarbonate is introduced into the foaming agent container **73.** When the foaming agent container **73** is fitted into the foaming agent path **7c,** the pushing bar **7f** pushes the closing plug **75** into the mixing space **7a** to allow the communication port **7e** to communicate with the mixing space **7a.** This causes the foaming agent to be mixed with the liquid component of the gas-liquid mixture, generating foam. Upon the foam generation, the corrugated pipe **72** extends so that the gas-liquid mixture is filled in the mixing space **7a** and the corrugated pipe **72.**

While, when the closing valve **7d** is opened and the corrugated pipe **72** is pushed under the condition that the insertion jig **3** is inserted in the intestine **12,** the intestine cleansing agent of the gas-liquid mixture is injected into the intestine **12** through the insertion jig **3.**

Thereafter, the operation for excreting the contents of the intestine **12** is performed likewise Working Example 1 or Working Example 2. The other constitution, operation, and effects are the same as in Working Example 1 and Working Example 2.

### <Working Example 4>

Working Example 4 of the present invention will be described next in detail with reference to the drawing.

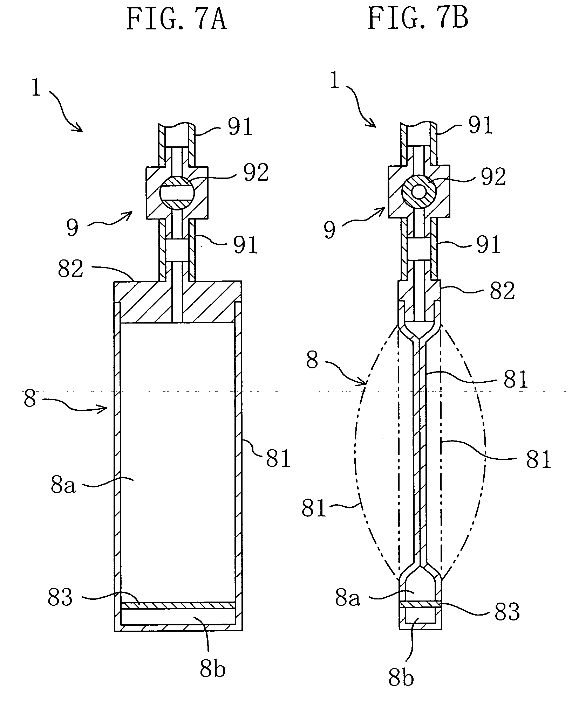

As shown in FIG. 7, a simple intestine cleaning apparatus **1** is employed rather than the intestine cleaning apparatus **1** of Working Example 1, and is of generally-called disposable type.

The intestine cleaning apparatus **1** includes a supply mechanism **8** and an insertion jig **9.** The supply mechanism **8** includes a bag body **81** and a connection plug **82.** The bag body **82** includes at the inside thereof a division wall **83** to form an upper chamber **8a** and a lower chamber **8b.** The bag body **81** is formed of a flexible member such as a plastic film, a rubber sheet, and the like. The division wall **83** is made of a brittle material so as to be easily broken by external force.

The upper chamber **8a** stores an acid liquid as a liquid component of a gas-liquid mixture, such as supernatant liquor of yogurt and lactic acid. On the other hand, the lower chamber **8b** stores a foaming agent such as an aqueous solution of baking soda.

The insertion jig **9** includes a conduit **91,** and a closing valve **91** provided in the middle of the conduit **91.** The conduit **91** is in a flexible, long, and narrow form so as to be inserted into the intestine **12.** The connection plug **82** of the supply mechanism **8** is connected to the conduit **91.**

### -Cleaning Method-

Cleaning operation of the aforementioned intestine apparatus **1** and an intestine cleaning method will be described next.

First, the division wall **83** of the supply mechanism **8** is broken while the closing valve **92** of the insertion jig **9** is closed. For example, the division wall **83** is broken by crumpling the bag body **81** by a hand. The breakage causes the upper chamber **8a** and the lower chamber **8b** to communicate with each other, generating the gas-liquid mixture including foam. The thus generated gas-liquid mixture as the intestine cleaning agent is filled in the bag body **81.**

Then, after the insertion jig **9** is inserted into the intestine **12,** the closing valve **92** is opened and bag body **81** is pushed, whereby the intestine cleaning agent is injected into the intestine **12.**

The sequence of the above operation may be changed, namely, the insertion jig **9** is inserted into the intestine **12** first, the closing valve **92** is opened, and then, the division wall **83** is broken.

When the insertion jig **3** is taken away and a predetermined time period passes after the injection, defecation is performed. The other constitution, operation, and effects are the same as in Working Example 1 or Working Example 2.

It is noted that the upper chamber **8a** may store a to-be-injected liquid containing a viscosity improver such as methylcellulose and the like in addition. In this case, in associating with the foam generation, an effective substance that lactic acid bacteria produce is injected into the intestine **12** together.

### <Working Example 5>

Working Example 5 of the present invention will be described next in detail with reference to the drawing.

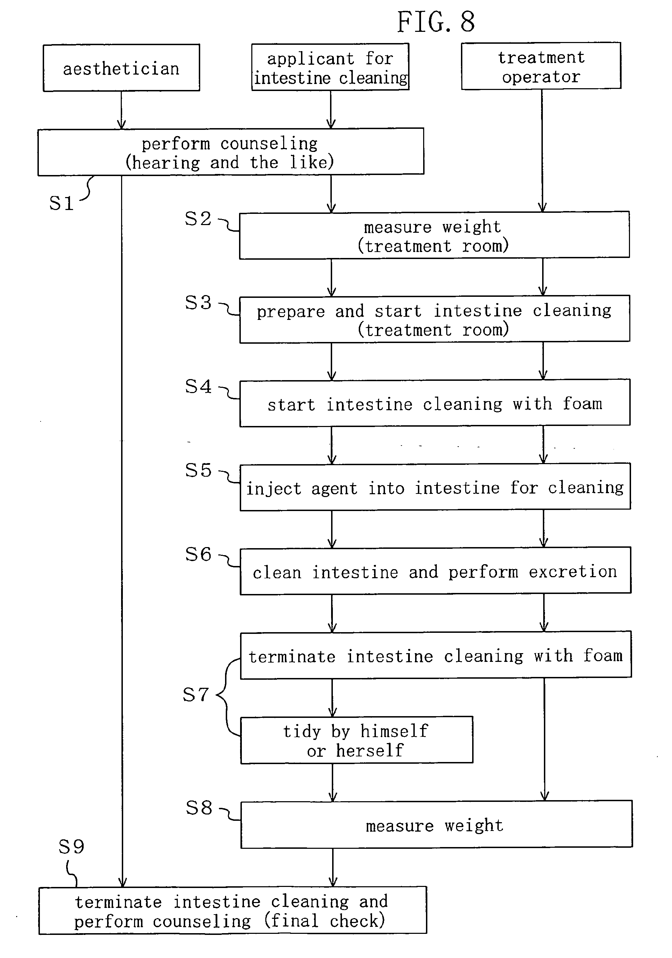

As shown in FIG. **8,** the present working example relates to intestine cleaning services using, for example, the intestine cleaning apparatus **1** in Working Example 1.

On example of the business model and the sequence thereof will be descried below.

For example, in an intestine cleaning station established in a town, services including explanation of dietary lifestyle habits are provided to general public.

### S 1. Counseling

An applicant for intestine cleaning formulates a personal file and put his or her health condition and lifestyle habits into an examination recipe. An aesthetician examines his or her present defecation condition based on the health examination recipe. The details and method of the cleaning treatment is explained at this counseling to ask for understanding and cooperation of the applicant for intestine cleaning.

### S2. Weight Measurement

A treatment operator introduces the applicant to a treatment room (a compartment) to let him or her change his or her clothes to an exclusive gown. After the applicant urinates at a toilet in the treatment room, his or her weight is measured.

### S3. Start of Intestine Cleaning

Before the cleaning, the applicant for intestine cleaning lies on his or her back on a treatment table. The intestine cleaning operator massages pathways of the applicant's entire intestine. Then, the intestine cleaning (comfortable cleaning with foam) is performed. The applicant's chest and below the chest are covered with a soft blanket.

### S4. Start of Comfort Cleaning with Foam

The applicant for intestine cleaning takes a Sims's position with lying on the left side and flexing the right knee toward the chest. The intestine cleaning operator applies a lubricant onto a personal-use insertion jig **3** for the intestine **12** and inserts it softly up to about 5 to 6 cm into the rectum of the applicant for intestine cleaning. The discharge pipe **41** is fitted to the insertion jig **3,** and then, the applicant for intestine cleaning takes a position with lying on the back again.

### S5. Injection into Intestine 12 for Cleaning

The foam-state intestine cleaning agent injected in the rectum flows through the descending colon to the ascending colon. The foam-state intestine cleaning agent to be injected, which is kept controlled at a temperature of about 37 °C by the apparatus of the present invention, flows into the intestine **12** slowly and softly. The cycle of the injection is about 5 minutes.

### S6. Intestine Cleaning and Excretion

The foam-state intestine cleaning agent flowing in the intestine **12** and fluidizing the detained feces is flown out through the discharge pipe **41** together with excrement in the intestine **12.** This cycle of the injection, cleaning, and discharge is repeated plural times to thoroughly clean the entire intestine.

### S7. End of Comfort Cleaning with Foam

Finally, the applicant excretes the residual intestine cleaning agent and feces in the exclusive toilet installed in the treatment room and tidies.

### S8. Weight Measurement

The intestine cleaning operator measures the weight of the applicant for intestine cleaning and records it.

### S9. Counseling after Intestine Cleaning

The aesthetician checks the health condition of the applicant for intestine cleaning and terminates the treatment. Thereafter, the applicant for intestine cleaning and the like stores his or her personal file into a cabinet at a cashier hall, pays the treatment fee, and goes home.

### <Embodiment 1>

Concrete examples of the intestine cleaning agent of the present invention will be described next.

Each aqueous solution containing at least any one of pectin, polyvinyl alcohol, methylcellulose, carboxy methyl cellulose, sodium polyacrylate, albumin, sugar ester, glycerin fatty acid ester, kudzu, saccharose, and a commercially available neutral detergent was used.

With the use of the above materials, evaluation of size, strength, and lifetime of foam was performed at: (1) foam formation by a mixer; (2) foam generation by a foam generator and injection into a pipe; (3) introduction of dry ice into a solution and foam flowing in connected a pipe; and (4) introduction of CO₂ foam generated by tartaric acid and baking soda into the solution.

Sufficient foam formation was attained with the use of methylcellulose and the commercially available neutral detergent. Also, a little amount of foam was formed with the use of polyvinyl alcohol.

Sole use of sugar ester or polyglycerol ester results in satisfactory foam formation.

Further, a liquid mixture of methylcellulose and sugar ester or polyglycerol ester attained sufficient foam formation, which suggests equivalent foam formation in a model intestine.

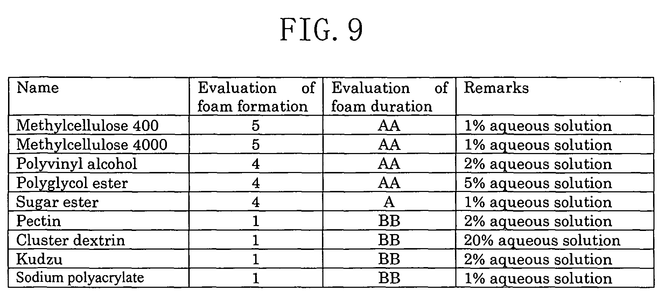

The obtained results were evaluated according to the following evaluation standards to obtain the results shown in FIG. **9.** Wherein, point 5 to point 1 as the evaluation standards for foam formation in FIG. **9** indicate: a large amount of foam generation (point 5); a middle amount of foam formation (point 4); a little amount of foam formation (point 3); a slight amount of foam formation (point 2); and little or no foam formation (point 1). In addition, the marks AA, A, and BB as the evaluation standards for the foam duration in FIG. **9** indicate: duration over 15 minutes (AA); duration within 15 minutes (A); and duration within 5 minutes (BB).

### <Embodiment 2>

Each of the following additives to excreted feces was tested on fluidization of hardened feces in the present invention: methylcellulose; sugar ester; polyglycerol ester; polyvinyl alcohol; a supernatant liquor of yogurt; cluster dextrin; pectin; and the like. Every case where any solution was added except sodium bicarbonate showed dispersion and fluidization of the hardened feces. Especially, rapid fluidization was attained with the use of sugar ester or polyglycerol ester.

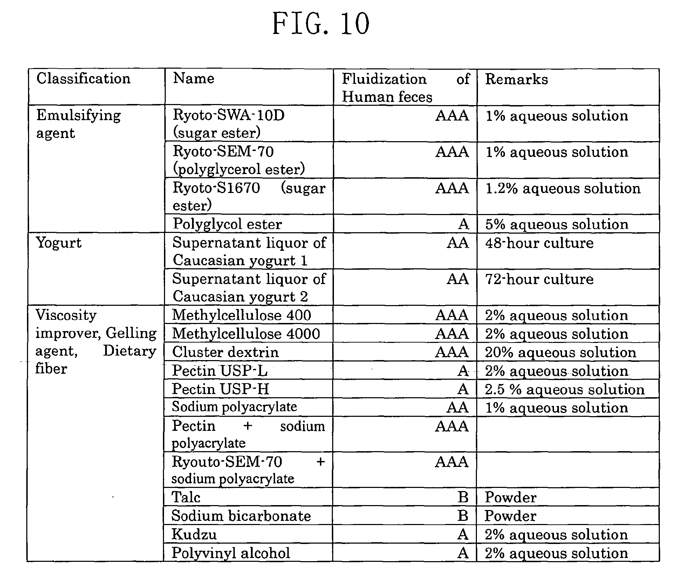

The results are indicated in FIG. **10.** The marks AAA, AA, A, and B as the evaluation standards for fluidization of human excreted feces in FIG. **10** indicate: immediate fluidization (AAA); fluidization within 5 minutes (AA); no satisfactory fluidization within 5 minutes (A); and no fluidization (B).

### <Embodiment 3>

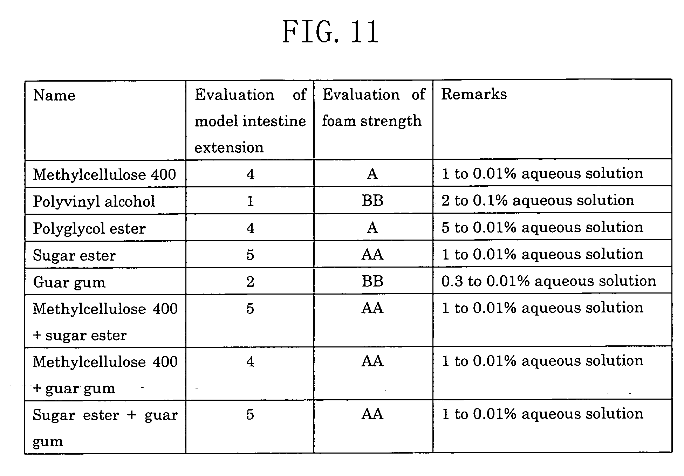

A soft tube of vinyl chloride having a total length of 1.5 m and an inner diameter of 30 mm was prepared. A foam-state gas-liquid mixture of CO₂ and at least one of solutions selected from methylcellulose, sugar ester, polyglycerol ester; polyvinyl alcohol, and guar gum was released to the vinyl tube, and extension and expansion by foam within the tube and the foam strength were evaluated. The results are indicated in FIG. **11.**

Sufficient expansion of the model intestine **12** by foam was observed with the use of each solution of sugar ester, polyglycerol ester, methylcellulose, and the like even when they were diluted. Sole use of guar gum attained insufficient foam but was useful for increasing the strength of the foam formed of the other materials.

The results are indicated in FIG. **11.** Point 5 to point 1 as the evaluation standards for extension of the model intestine **12** in FIG. **11** indicate: high level extension by foam (point 5); middle level extension by foam (point 4); a little amount of extension by foam (point 3); no extension by foam (point 2); and separation of liquid (point 1).

Further, the marks AA, A, and BB as the evaluation standards for strength of foam in the model intestine **12** indicate: formation of a foam layer over 20 cm (AA); formation of a foam layer from 20 to 10 cm (A); and formation of a foam layer less than 10 cm (BB).

It is noted that each working example and each embodiment describe the case addressing an intestine as an organic lumen but the present invention may address the other organic lumens. The organic lumens includes: nasal cavities; auricular cavities; oral cavities; tracheae; bronchi; esophagi; stomachs; duodena; small intestines; gallbladders; bile ducts; pancreatic ducts; myelonic ducts; subdural cavities; pleuroperitoneal cavities; pericardial cavities; mediastinal cavities; renal pelvises; urethras; vesicourethral canales; uterovaginal cavities; joint cavities; abscess cavities; infection focuses; cysts; blood vessels; and the like.

Further, each working example and each embodiment direct to a foam-state substance as the gas-liquid mixture, but the gas-liquid mixture in the present invention may be in a vapor state that a liquid component floats in a gas component. In short, the gas-liquid mixture in any state can be used only if a gas component and a liquid component are mixed.

### Industrial Applicability

As described above, the organic lumen cleaning agent, the organic lumen cleaning apparatus, and the organic lumen cleaning method according to the present invention are useful for cleaning organic lumens, and especially useful for excretion of detained feces, thereby being suitable for various defecation impairment.

## Claims

1. An organic lumen cleaning agent comprising:
a gas-liquid mixture obtained by mixing at least a gas component and a liquid component.

2. The organic lumen cleaning agent of Claim 1, wherein
the gas-liquid mixture is in a vapor state that the liquid component floats in the gas component.

3. The organic lumen cleaning agent of Claim 1, wherein
the gas-liquid mixture is in a foam state that the gas component is encapsulated in the liquid component.

4. The organic lumen cleaning agent of Claim 1, wherein
the gas-liquid mixture has a property of fluidizing or dispersing contents of an organic lumen.

5. The organic lumen cleaning agent of Claim 1, wherein
the gas component of the gas-liquid mixture is a gas including at least one of air, oxygen, nitrogen, carbon dioxide, argon, and helium, and
the liquid component of the gas-liquid mixture is a liquid of any of an aqueous solution, an oil-based liquid, a water- and oil-based liquid, and a mixed solution of a water-based material and an oil-based material.

6. The organic lumen cleaning agent of Claim 1, wherein
a solute component of the gas-liquid mixture is at least one selected from the group of: celluloses; uronic acids; starches and starch-derived products; dextrins; lactobacillary yogurt and yogurt-derived products; mucopolysaccharides; rubbers; polyvinyl alcohol; polyvinyl pyrrolidone; sugar ester; polyglycerol ester; polyethylene glycol; glycerin; kudzu; seaweeds and seaweeds-derived products; natural oils and synthetic oils; detergents; herbal aromatic substances; natural bactericides and synthetic bactericides; deodorizers; oligosaccharide and monosaccharides; electrolytes; inorganic alkalis; and carboxylic acids.

7. The organic lumen cleaning agent of Claim 1, wherein
a raw material of the gas-liquid mixture includes a CO₂ generating agent and at least one of bacteria, bacteria-derived products; and carboxylic acids.

8. The organic lumen cleaning agent of Claim 1, wherein
the gas-liquid mixture contains at least one of a biological origin and a physiological modifier.

9. An organic lumen cleaning apparatus comprising:
insertion means to be inserted into an organic lumen; and
cleaning agent means, which stores an organic lumen cleaning agent composed of a gas-liquid mixture obtained by mixing at least a gas component and a liquid component or which generates an organic lumen cleaning agent from a raw material component of the gas-liquid mixture, for supplying the organic lumen cleaning agent to the insertion means.

10. The organic lumen cleaning apparatus of Claim 9, further comprising:
discharge means connected to the insertion means for recovering contents of the organic lumen which is excreted from the organic lumen.

11. The organic lumen cleaning apparatus of Claim 9, further comprising:
volume control means connected to the connection means for controlling a supply amount of the organic lumen cleaning agent.

12. The organic lumen cleaning apparatus of Claim 9, wherein
the cleaning agent means includes a flexible container, and a division wall which is provided within the container, which defines the inside of the container into plural, and which is capable of being broken.

13. The organic lumen cleaning apparatus of Claim 9, wherein
the gas-liquid mixture is in a foam state that the gas component is encapsulated in the liquid component.

14. An organic lumen cleaning method using:
insertion means to be inserted into an organic lumen; and
cleaning agent means, which stores an organic lumen cleaning agent composed of a gas-liquid mixture obtained by mixing at least a gas component and a liquid component or which generates an organic lumen cleaning agent from a raw material component of the gas-liquid mixture, for supplying the organic lumen cleaning agent to the insertion means, the method comprising the steps of:
inserting the insertion means into an organic lumen;
injecting the organic lumen cleaning agent from the cleaning agent means through the insertion means.

15. The organic lumen cleaning method of Claim 14, further comprising the step of:
recovering contents of the organic lumen which is excreted from the organic lumen to discharge means connected to the insertion means after cleaning by injecting the organic lumen cleaning agent into the organic lumen.

16. The organic lumen cleaning method of Claim 14, wherein
a supply amount of the organic lumen cleaning agent is controlled by volume control means connected to the connection means.

17. The organic lumen cleaning method of Claim 14, wherein
the gas-liquid mixture is in a foam state that the gas component is encapsulated in the liquid component.

18. The organic lumen cleaning method of Claim 17, further comprising the step of:
administering an antifoaming agent to the organic lumen in a cleaning process after injecting the organic lumen cleaning agent to the organic lumen.
